# EUROPEAN PATENT APPLICATION

(11) **EP 4 635 438 A1**
(43) Date of publication of application: **22.10.2025**
(21) Application number: 23920301.1
(22) Date of filing: 07.02.2023
(51) Int. Cl.: A61B 18/12, A61B 18/14

(54) **BALLOON ELECTRODE CATHETER, BALLOON ELECTRODE ASSEMBLY, AND FLEXIBLE CIRCUIT OF BALLOON ELECTRODE ASSEMBLY**

(71) Applicant: Enchannel Medical Guangzhou Inc., Guangzhou, Guangdong 510700 (CN)
(72) Inventor: MO, Zhidai, Guangzhou, Guangdong 510700 (CN); HUANG, Long, Guangzhou, Guangdong 510700 (CN); CHEN, Hao, Guangzhou, Guangdong 510700 (CN); SONG, Qing, Guangzhou, Guangdong 510700 (CN); FENG, Jun, Guangzhou, Guangdong 510700 (CN)
(74) Representative: Manitz Finsterwald Patent- und Rechtsanwaltspartnerschaft mbB
(86) International application number: PCT/CN2023/074771
(87) International publication number: WO 2024/164141

(57) **Abstract**

The present invention relates to a balloon electrode catheter, in particular to a flexible circuit for a balloon electrode assembly in a balloon electrode catheter. The flexible circuit comprises an insulating substrate, electrode pads, and traces; wherein the insulating substrate is configured to attach to the balloon of the balloon electrode assembly and to conform to curvature changes during expansion and contraction of the balloon body; the electrode pads are fixed to the insulating substrate for generating a pulse electric field; the trace is located inside the insulating substrate for connecting the electrode pads to corresponding circuits; and the traces include at least one branch trace, the branch trace being arranged side by side with at least one electrode pad along the extended surface of the insulating substrate and being connected to another electrode pad, such that electrical connection of corresponding electrode pad is achieved by means of the branch trace. The above solution is conducive to reducing the hardness of the flexible circuit, making it easier for the flexible circuit to deform when subjected to resistance in the process of entering and exiting the introducer sheath, avoiding serious interference with the sheath, and facilitating the entry and exit of the balloon electrode assembly into and out of the sheath.

## Description

### TECHNICAL FIELD

The present disclosure relates to balloon electrode catheters, and more particularly to a flexible circuit for a balloon electrode assembly used in such catheters.

### BACKGROUND OF THE DISCLOSURE

The balloon electrode catheter generally comprises a tubular body and a balloon electrode assembly disposed at the distal end of the tubular body. The balloon electrode catheter can be advanced through an introducer sheath inserted into the body to a target site region within the body. Thereafter, a fluid (e.g., gas or liquid) is introduced through the proximal end of the balloon electrode catheter to inflate and expand the distal balloon, thereby causing a flexible circuit positioned on the balloon to deploy into a predetermined shape. This deployment positions electrodes on the flexible circuit in contact with the target tissue within the body. Subsequently, electrical energy is delivered through the electrodes to achieve the therapeutic effect. A typical application of the balloon electrode catheter utilizes pulsed electric field energy for the treatment of atrial fibrillation. The fundamental principle involves applying high-voltage, short-duration pulses to the tissue via pairs of electrodes, inducing irreversible electroporation (IRE) in tissue cells, thereby which results in necrosis or apoptosis of the target tissue. One advantage of pulsed electric field ablation (PFA) compared to radiofrequency ablation (RF) for atrial fibrillation ablation is its non-thermal mechanism, meaning minimal to no temperature elevation occurs during ablation. Consequently, PFA can mitigate complications such as tissue charring, thrombosis, and pulmonary vein stenosis (PVS) associated with thermal overheating.

The flexible circuit for the balloon electrode assembly generally includes an insulating substrate formed from an insulating material, electrodes attached to the insulating substrate, and traces for connecting the electrodes to corresponding circuitry. The traces are embedded within the insulating substrate and are arranged on a different layer relative to the electrodes, thereby electrically connecting the electrodes to the corresponding circuit.

Although the flexible circuit is flexible and capable of deformation, its inherent rigidity makes insertion and withdrawal of the deflated balloon electrode assembly into and out of the sheath difficult.

### SUMMARY OF THE DISCLOSURE

A primary objective of the present disclosure is to overcome the technical problem of difficulty in inserting prior art balloon electrode assemblies into and withdrawing them from an introducer sheath.

A flexible circuit for a balloon electrode assembly is provided according to a first aspect of the present disclosure.

The flexible circuit for a balloon electrode assembly includes:
an insulating substrate, configured to attach to a balloon body of the balloon electrode assembly and to conform to curvature changes during the expansion and contraction of the balloon body;
electrode pads, fixed on the insulating substrate and configured to generate a pulsed electric field; and
traces, disposed within the insulating substrate and configured to connect the electrode pads to a corresponding circuit;
wherein the traces comprises at least one branch traces that is disposed side by side with at least one electrode pad along an extended surface of the insulating substrate and connected to another electrode pad, such that said electrode pads are electrically connected through the branch traces.

In some embodiments, the insulating substrate comprises at least one branch portion configured to accommodate the branch portion therein, and at least a section of the branch portion is detached from a main body portion of the insulating substrate.

In some embodiments, at least one end of the branch portion along its length comprises an oblique transition section, serving as a guide structure for guiding the branch portion into and out of a corresponding introducer sheath.

In some embodiments, the oblique transition section has a convexly arcuate shape.

In some embodiments, the thickness of the branch portion exceeds that of the main body portion of the insulating substrate.

In some embodiments, the electrode pad comprises a first electrode pad, a second electrode pad and a third electrode pad that are arranged sequentially along a direction from a proximal end to a distal end of the flexible circuit, at least two branch traces are connected to a side of the first electrode pad opposite to the second electrode pad, said branch traces being respectively located on opposite widthwise sides of the flexible circuit, and respectively connected to the second electrode pad and the third electrode pad.

In some embodiments, the traces and the electrode pads are free of overlap along a thickness direction of the flexible circuit.

A balloon electrode assembly is provided according to a second aspect of the present disclosure.

The balloon electrode assembly includes:
a balloon body capable of being expanded and deflated; and
a flexible circuit, wherein the flexible circuit includes:
   an insulating substrate, configured to attach to a balloon body of the balloon electrode assembly and to conform to curvature changes during expansion and contraction of the balloon body;
   electrode pads, fixed on the insulating substrate and configured to generate a pulsed electric field; and
   traces, disposed within the insulating substrate and configured to connect the electrode pads to a corresponding circuit;
   wherein the traces comprise at least one branch trace that is disposed side by side with at least one electrode pad along an extended surface of the insulating substrate and connected to another electrode pad, such that said electrode pads are electrically connected through the branch trace.

In some embodiments, the insulating substrate comprises at least one branch portion configured to accommodate the branch portion therein, and at least a section of the branch portion is detached from a main body portion of the insulating substrate.

In some embodiments, at least one end of the branch portion along its length comprises an oblique transition section, serving as a guide structure for guiding the branch portion into and out of a corresponding introducer sheath.

In some embodiments, the oblique transition section has a convexly arcuate shape.

In some embodiments, the thickness of the branch portion exceeds that of the main body portion of the insulating substrate.

In some embodiments, the electrode pad comprises a first electrode pad, a second electrode pad and a third electrode pad that are arranged sequentially along a direction from a proximal end to a distal end of the flexible circuit, at least two branch traces are connected to a side of the first electrode pad opposite to the second electrode pad, said branch traces being respectively located on opposite widthwise sides of the flexible circuit, and respectively connected to the second electrode pad and the third electrode pad.

In some embodiments, the traces and the electrode pads are free of overlap along a thickness direction of the flexible circuit.

In some embodiments, the branch portion of the insulating substrate for carrying the branch traces is bonded to the surface of the balloon body.

A balloon electrode catheter is provided according to a third aspect of the present disclosure.

The balloon electrode catheter includes:
a tubular body; and
a balloon electrode assembly, connected to a distal end of the tubular body;
wherein the balloon electrode assembly comprises:
   a balloon body capable of being expanded and deflated; and
   a flexible circuit, wherein the flexible circuit comprises:
      an insulating substrate, configured to attach to a balloon body of the balloon electrode assembly and to conform to curvature changes during expansion and contraction of the balloon body;
      electrode pads, fixed on the insulating substrate and configured to generate a pulsed electric field; and
      traces, disposed within the insulating substrate and configured to connect the electrode pads to a corresponding circuit;
      wherein the traces comprise at least one branch trace that is disposed side by side with at least one electrode pad along an extended surface of the insulating substrate and connected to another electrode pad, such that said electrode pads are electrically connected through the branch trace.

In some embodiments, the insulating substrate comprises at least one branch portion configured to accommodate the branch portion therein, and at least a section of the branch portion is detached from a main body portion of the insulating substrate.

In some embodiments, at least one end of the branch portion along its length comprises an oblique transition section, serving as a guide structure for guiding the branch portion into and out of a corresponding introducer sheath.

In some embodiments, the oblique transition section has a convexly arcuate shape.

In some embodiments, the thickness of the branch portion exceeds that of the main body portion of the insulating substrate.

In some embodiments, the electrode pad comprises a first electrode pad, a second electrode pad and a third electrode pad that are arranged sequentially along a direction from a proximal end to a distal end of the flexible circuit, at least two branch traces are connected to a side of the first electrode pad opposite to the second electrode pad, said branch traces being respectively located on opposite widthwise sides of the flexible circuit, and respectively connected to the second electrode pad and the third electrode pad.

In some embodiments, the traces and the electrode pads are free of overlap along a thickness direction of the flexible circuit.

In some embodiments, the branch portion of the insulating substrate for carrying the branch traces is bonded to a surface of the balloon body.

The beneficial effects of the disclosure:
According to the flexible circuit for the balloon electrode assembly mentioned above, the provision of branch traces enables the corresponding electrode pads to bypass other electrode pads via these traces. This arrangement thereby reduces the number of traces overlapping with the electrode pads in the thickness direction of the flexible circuit, or prevents the traces and electrode pads from being stacked along the thickness direction. Consequently, the hardness of the flexible circuit is reduced, allowing it to deform more readily when encountering resistance during insertion into and withdrawal from the introducer sheath. This helps avoid undue interference with the sheath and facilitates the advancement and retraction of the balloon electrode assembly within the introducer sheath.

### BRIEF DESCRIPTION OF THE DRAWINGS

FIG. 1 is a schematic view of a balloon electrode assembly in Embodiment 1 of the present disclosure;
FIG. 2 is a sectional view of the assembly in FIG. 1;
FIG. 3 is a schematic view of a balloon electrode assembly in Embodiment 2 of the present disclosure; and
FIG. 4 is the schematic view of a flexible circuit for a balloon electrode assembly of a prior art design.

### List of Reference Numerals:

- 10: tubular body;
- 20: balloon electrode assembly;
- 21: balloon body;
- 22: flexible circuit;
- 221: insulating substrate;
- 222: electrode pad;
- 223: trace;
- 224: main body portion;
- 225: branch portion;
- 226: oblique transition section;
- 227: branch trace;
- 228: adhesive.

### DETAILED DESCRIPTION

Specific embodiments of the present disclosure will now be described in detail with reference to the accompanying drawings. Similar or related components in different embodiments are labeled with associated reference numerals. The following embodiments include detailed descriptions to facilitate understanding of the present disclosure. However, those skilled in the art will readily recognize that certain features may be omitted under specific circumstances or substituted by other components, materials, or methods. In some instances, certain operations related to the present disclosure are not explicitly described or illustrated herein. This intentional exclusion is intentional to avoid obscuring the core technical solutions of the present disclosure. For those skilled in the art, a complete understanding of these operations can be attained through the descriptions provided in this specification and general technical knowledge in the art.

Additionally, the features, operations, or characteristics described in the specification may be combined in any suitable manner to form various embodiments. Similarly, steps or actions in the method descriptions may be reordered or modified in ways that would be obvious to those skilled in the art. Therefore, the sequences presented in the specification and drawings are intended solely to clarify the description of specific embodiments and do not imply mandatory orderings, unless explicitly stated that a particular sequence is required.

The numerical designations assigned to components in this specification, such as 'first,' 'second,' or similar ordinal terms, serve solely to distinguish described objects and carry no inherent sequential or technical implications. Furthermore, the terms 'connected' and 'coupled' as used herein encompass both direct and indirect connection (coupling), unless explicitly stated otherwise.

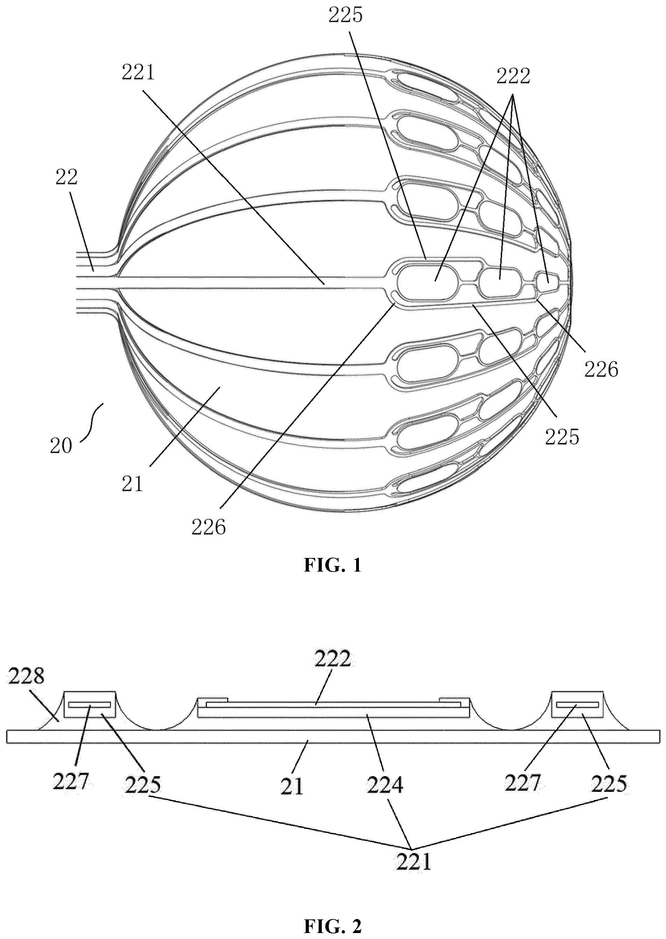

Embodiment 1 of the balloon electrode assembly in the present disclosure:
Referring to FIGS. 1 and 2, the balloon electrode assembly 20 includes a balloon body 21 and a flexible circuit 22.

In a preferred arrangement, the balloon body 21 is generally made from polymer materials such as nylon, PEBAX, or PET. The balloon body 21 expands when inflated with a gas and contracts when deflated. It defines a proximal end, a distal end, a longitudinal axis extending between the proximal and distal ends, and a circumferential direction around the longitudinal axis. It should be noted that in the medical field and for devices such as balloon electrode catheters, the term "proximal end" conventionally refers to the end close to an operator manipulating the device during use, and the term "distal end" refers to the end farther from the operator during use. Alternatively, the balloon body 21 may be filled with other fluids, such as water or liquid medication.

In a preferred arrangement, the flexible circuit 22 comprises a plurality of circuit strips. Each strip is uniformly distributed along the circumference of the balloon body 21 and corresponds to a generatrix on the expanded balloon body 21. The curved surface pattern can be seen as the track of a moving line, and the moving line forming the curved surface is the generatrix. For example, for the spherical surface formed after inflation of the balloon body 21, the intersection line between a plane passing through the axis of the balloon body 21 and the surface of the balloon body 21 is the generatrix. The number of circuit strips in this embodiment is twelve. In other embodiments, the number of circuit strips may also be increased or decreased as needed. Each circuit strip extends from the proximal end to the distal end of the balloon body 21. Both ends along the length of each circuit strip define its proximal and distal ends, respectively. To facilitate circumferential positioning of each circuit strips, the distal end of each circuit strip is integrally connected to a common annular circuit strip. In other embodiments, each circuit strip can be independent of the others. Additionally, in other embodiments, the distal end of each circuit strip may be positioned at a distance from the distal end of the balloon body 21.

Each circuit strip includes an insulating substrate 221, electrode pads 222, and traces. The insulating substrate 221 is configured to attach to the balloon body 21 so that the circuit strip can conform to curvature changes with the expansion and deflation of the balloon body 21. Of course, the insulating substrate 221 is a flexible substrate with a predetermined thickness to enable bending deformation. The electrode pads 222 are fixed on the outer surface of the insulating substrate 221, typically by bonding. The surfaces of the electrode pads 222 are exposed on the circuit strip for generating a pulsed electric field. The traces are formed within the insulating substrate 221. Copper is commonly used for both the traces and the electrode pads 222 to ensure effective circuit conduction. The traces extend along the circuit strip. The traces extend along the circuit strip, with their proximal ends reaching the distal end of the corresponding tubular body 10 where they are welded to corresponding micro-cables. These micro-cables run along the tubular body 10 to a handle disposed at its proximal end of the tubular body 10 and are welded to a connector on the handle. In other embodiments, the electrode pads 222 can also be formed on the insulating substrate 221 by etching, which is a mature process in the field of circuit board manufacturing and will not be described in detail here.

In a preferred arrangement of this embodiment, each circuit strip includes three electrode pads 222, designated as the first , second, and third electrode pads. The three electrode pads 222 are arranged sequentially from the proximal end to the distal end of the flexible circuit 22, and are located on the distal half of the balloon body 21. This positioning enables the electrode pads 222 to contact target biological tissue as the balloon electrode catheter is advanced forward. Additionally, to adapt to the varying circumferential space along the length of the expanded balloon body 21, the overall width of the group of electrode pads 222 decreases towards the distal end of the balloon body 21. Furthermore, the width of each individual electrode pad 222 also tapers from its proximal end to its distal end along the circuit strip. In other embodiments, the number of electrode pads 222 on each circuit strip may be increased or decreased as required.

The traces are located inside the insulating substrate 221 and are used to connect each electrode pad 222 to its corresponding circuit. In a preferred arrangement, the insulating substrate 221 includes two branch portions 225, which extend from the side of the first electrode pad 222 away from the second electrode pad 222, and are respectively located on opposite lateral sides of the flexible circuit 22. The distal ends of the two branch portions 225 are connected to the insulating substrate 221 corresponding to the second electrode pad 222 and the third electrode pad 222, respectively. The traces include branch traces 227 formed within each branch portion 225. Except for the first electrode pad 222, the other two electrode pads 222 are electrically connected to their corresponding circuits via the branch traces 227. In a preferred arrangement, during assembly, the branch portions 225 are bonded and fixed onto the surface of the balloon body 21 using an adhesive 228. The adhesive 228 used to bond the branch portions 225 can be the same type as the adhesive 228 between the balloon body 21 and the main body portion 224 of the insulating substrate 221, for example, epoxy resin, acrylic resin, polyurethane glue, etc. The use of the branch portions to route the branch traces prevents excessive widening of the insulating substrate, thereby facilitating insertion of the flexible circuit into the introducer sheath. In other embodiments, when the insulating substrate 221 has more than two branch portions 225, the branch portions 225 can all be located on the same side of the main body portion 224.

In a preferred arrangement, the proximal and distal ends of each branch portion 225 are both provided with an oblique transition section 226 featuring a convexly arcuate shape. This arcuate transition facilitates the insertion and withdrawal of both the branch portions 225 and the balloon electrode assembly 20 into/from the introducer sheath. The convexly arcuate shape of these oblique transition sections 226 promotes smooth bending of the branch traces 227 embedded within them, thereby protecting the branch traces from damage due to stress concentration. In other embodiments, the oblique transition sections 226 may alternatively be of an inclined linear shape.

In a preferred arrangement, due to the reduced dimensions of the branch portion 225 of the insulating substrate 221, its thickness is designed to be greater than that of the main body portion 224 of the insulating substrate 221 to ensure its structural strength and to prevent damage to the internal traces caused by excessive bending.

When in use, the balloon body 21 in the balloon electrode assembly 20 is controlled to deflate, causing it to collapse. The circuit strips on the balloon body 21 move closer to each other and fold into a circumferential arrangement adjacently aligned or partially overlapped. The balloon electrode catheter may then be operated to advance via the tubular body 10 into the corresponding introducer sheath. After the balloon electrode assembly 20 exits the distal end of the introducer sheath, the balloon body 21 in the balloon electrode assembly 20 is inflated to achieve a predetermined shape and size. This simultaneously deploys the circuit strips into the target configuration, allowing the electrode pads 222 to conform to the target biological tissue for pulse electric field ablation. After ablation, the balloon body 21 in the balloon electrode assembly 20 is controlled to deflate again, retracting the circuit strips to enable withdrawal from the introducer sheath.

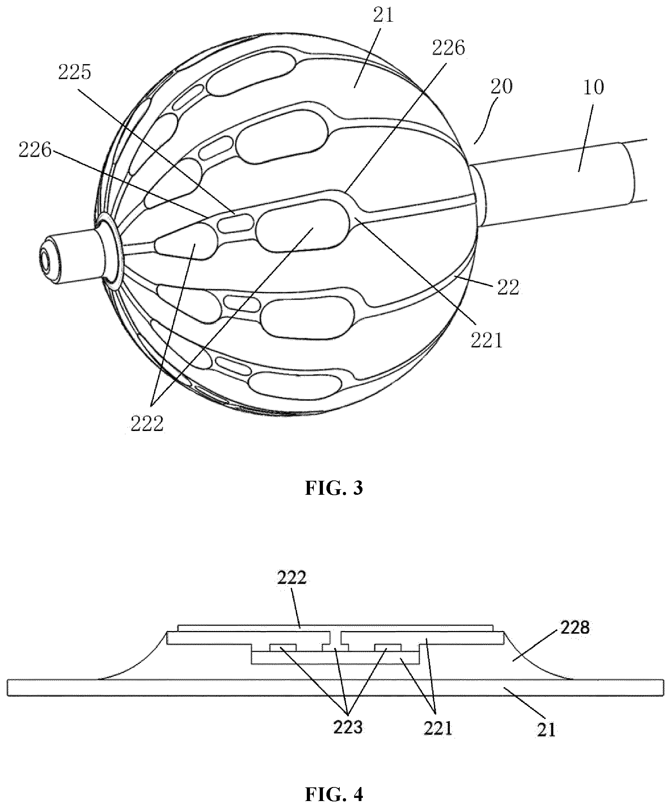

Since the traces connected to the first electrode pad 222 can lie directly within the same electrode extension plane (thus not shown in the sectional view), and the traces connected to the second and third electrode pads 222 can also lie within the same plane as each electrode pad 222 via the branch portion 225 of the insulating substrate 221, there is no overlap between the traces and the electrode pads 222 along the thickness direction of the flexible circuit 22. This allows effective reduction in the thickness of the insulating substrate 221, thereby enabling control over the hardness of the flexible circuit 22. As a result, when subjected to resistance during insertion into or withdrawal from the introducer sheath, the flexible circuit 22 can deform more easily, avoiding significant interference with the introducer sheath and facilitate the entry and exit of the balloon electrode assembly 20. Furthermore, the oblique transition sections 226 at both longitudinal ends of the branch section 225 serve to guide the balloon electrode assembly 20, further facilitating the insertion and withdrawal of the balloon electrode assembly 20 relative to the introducer sheath. Please refer to FIG. 4. Prior to the improvement, the traces 223 and their corresponding electrode pads 222 are stacked along the thickness direction of the flexible circuit 22. The insulating substrate 221 includes an insulating protective layer on both the upper and lower sides of the traces 223. This configuration results in increased thickness and hardness of the flexible circuit 22, preventing it from flexing sufficiently in response to external forces encountered during insertion or withdrawal, making the access of the sheath difficult.

Embodiment 2 of the balloon electrode assembly in the present disclosure:
Please refer to FIG. 3. The difference between this embodiment and Embodiment 1 is that in this embodiment, each circuit strip is provided with two electrode pads 222, whereas only one branch portion 225 is provided. The section of the branch portion 225 corresponding to a gap between the two electrode pads 222 is detached from the main body portion of the insulating substrate 221, whereas the remainder is connected to the main body portion of the insulating substrate 221. This design facilitates the positioning of the branch portion 225.

In the above embodiments, no traces are stacked with the electrode pads 222 along the thickness direction of the flexible circuit 22. In other embodiments, besides the branch traces 227 in the branch section 225, some traces may still be stacked with their corresponding electrode pads along the thickness direction of the flexible circuit 22. Due to the presence of the branch traces 227 (which eliminate the need for stacking themselves), the total number of traces overlapping the electrode pad can be reduced. This still reduces the hardness of the part of the flexible circuit 22 corresponding to the electrode pads, thus still facilitating the insertion and withdrawal of the balloon electrode assembly 20 relative to the introducer sheath.

Embodiments of the flexible circuit for a balloon electrode assembly in the present disclosure:
The flexible circuit for a balloon electrode assembly has the same structure as the flexible circuit 22 in any embodiment of the balloon electrode assembly 20, and details thereof are omitted herein.

Embodiments of a balloon electrode catheter in the present disclosure:
The balloon electrode catheter comprises a tubular body 10, a balloon electrode assembly 20, and a handle (not shown in the figure) as shown in FIG. 3. The proximal end of the tubular body 10 is connected to the handle, while the distal end of the tubular body 10 is connected to the balloon electrode assembly 20. The tubular body 10 is configured to advance the balloon electrode assembly 20 through a corresponding introducer sheath and deliver the balloon electrode assembly 20 to a designated site within a biological body. The balloon electrode assembly 20 has the same structure as the balloon electrode assembly in any embodiment of the balloon electrode assembly, and details thereof are omitted herein.

The specific examples employed above to illustrate the present disclosure are for the purpose of facilitating understanding and are not intended to limit the disclosure. Those skilled in the art to which the present disclosure pertains, without departing from the basic principles of the present disclosure, may make various simple deductions, modifications or substitutions.

## Claims

1. A flexible circuit for a balloon electrode assembly, comprising:
an insulating substrate, configured to attach to a balloon body of the balloon electrode assembly and to conform to curvature changes during expansion and contraction of the balloon body;
electrode pads, fixed on the insulating substrate and configured to generate a pulsed electric field; and
traces, disposed within the insulating substrate and configured to connect the electrode pads to a corresponding circuit;
wherein the traces comprise at least one branch trace that is disposed side by side with at least one electrode pad along an extended surface of the insulating substrate and connected to another electrode pad, such that said electrode pads are electrically connected through the branch trace.

2. The flexible circuit for a balloon electrode assembly according to claim 1, wherein the insulating substrate comprises at least one branch portion configured to accommodate the branch portion therein, and at least a section of the branch portion is detached from a main body portion of the insulating substrate.

3. The flexible circuit for a balloon electrode assembly according to claim 2, wherein at least one end of the branch portion along its length comprises an oblique transition section, serving as a guide structure for guiding the branch portion into and out of a corresponding introducer sheath.

4. The flexible circuit for a balloon electrode assembly according to claim 3, wherein the oblique transition section has a convexly arcuate shape.

5. The flexible circuit for a balloon electrode assembly according to any one of claims 2 to 4, wherein a thickness of the branch portion exceeds that of the main body portion of the insulating substrate.

6. The flexible circuit for a balloon electrode assembly according to any one of claims 1 to 4, wherein the electrode pad comprises a first electrode pad, a second electrode pad and a third electrode pad that are arranged sequentially along a direction from a proximal end to a distal end of the flexible circuit,
at least two branch traces are connected to a side of the first electrode pad opposite to the second electrode pad, said branch traces being respectively located on opposite widthwise sides of the flexible circuit, and respectively connected to the second electrode pad and the third electrode pad.

7. The flexible circuit for a balloon electrode assembly according to any one of claims 1 to 4, wherein the traces and the electrode pads are free of overlap along a thickness direction of the flexible circuit.

8. A balloon electrode assembly, comprising:
a balloon body capable of being expanded and deflated; and
the flexible circuit according to any one of claims 1 to 7.

9. The balloon electrode assembly according to claim 8, wherein the branch portion of the insulating substrate for carrying the branch traces is bonded to a surface of the balloon body.

10. A balloon electrode catheter, comprising:
a tubular body; and
a balloon electrode assembly, connected to a distal end of the tubular body;
wherein the balloon electrode assembly comprises:
a balloon body capable of being expanded and deflated; and
the flexible circuit according to any one of claims 1 to 7.
